# EUROPEAN PATENT SPECIFICATION

(11) **EP 1 223 985 B1**
(45) Date of publication and mention of the grant of the patent: **30.11.2005**
(21) Application number: 00968956.3
(22) Date of filing: 13.10.2000
(51) Int. Cl.: A61K 47/48, A61P 13/00, A61K 31/765

(54) **DIALYSIS SOLUTION INCLUDING POLYGLYCOL OSMOTIC AGENT**
DIALYSELÖSUNG DIE POLYGLYCOL ALS OSMOTISCHEN MITTELS ENTHÄLT
SOLUTION DE DIALYSE CONTENANT UN AGENT OSMOTIQUE POLYGLYCOL

(30) Priority: 15.10.1999 US 159810 P
(43) Date of publication of application: 24.07.2002
(73) Proprietor: DOW GLOBAL TECHNOLOGIES INC., Midland, Michigan 48674 (US)
(72) Inventor: SIMON, Jaime, Algleton, TX 77515 (US); STRICKLAND, Alan, D., Lake Jackson, TX 77566 (US); STROM, Robert, M., Midland, MI 48640 (US)
(74) Representative: Raynor, John
(86) International application number: PCT/US2000/028523
(87) International publication number: WO 2001/028544

(56) References cited:
- WO-A-98/17707
- US-A- 5 496 545
- FUJIMURA N ET AL: "Effect of free radical scavengers on diaphragmatic contractility in septic peritonitis." AMERICAN JOURNAL OF RESPIRATORY AND CRITICAL CARE MEDICINE, (2000 DEC) 162 (6) 2159-65. , XP000997217
- GALINANES M. ET AL: "PEG -SOD and myocardial protection: Studies in the blood- and crystalloid- perfused rabbit and rat hearts." CIRCULATION, (1992) 86/2 (672-682). , XP000997181
- FUJIMURA N ET AL: "Alteration in diaphragmatic contractility during septic peritonitis in rats: effect of polyethylene glycol -absorbed superoxide dismutase." CRITICAL CARE MEDICINE, (2000 JUL) 28 (7) 2406-14. , XP000997182
- DAVANKOV V ET AL: "Polymeric adsorbent for removing toxic proteins from blood of patients with kidney failure" JOURNAL OF CHROMATOGRAPHY. BIOMEDICAL APPLICATIONS,NL,ELSEVIER, AMSTERDAM, vol. 739, no. 1, February 2000 (2000-02), pages 73-80, XP004190288 ISSN: 0378-4347

## Description

Renal dialysis involves the diffusion of water and waste products, (e.g., urea, excess salts, toxins, impurities, etc.) from a patient's blood, through a semipermeable membrane, and into a dialysis solution. Dialysis most commonly takes one of two forms: hemodialysis involves contacting a portion of the patient's blood with a synthetic semipermeable membrane wherein water and waste products diffuse from the blood through the membrane and into a dialysis solution. The "cleansed" blood is then returned to the patient. Peritoneal dialysis involves infusing a dialysis solution into the patient's peritoneum. The peritoneum comprises a cavity surrounded by blood vessels and capillary beds allowing it to act as a natural semipermeable membrane. Water and waste products diffuse from the blood, through the peritoneum and into the dialysis solution, which is subsequently removed from the patient.

Dialysis solutions are typically aqueous solutions including electrolytes, bicarbonate buffer, and an osmotic agent, i.e., a constituent utilized to create an osmotic gradient between a patient's blood and the dialysis solution. The most commonly used osmotic agents include a carbohydrate containing osmotic agent such as glucose and dextrose.

There are several problems associated with peritoneal dialysis. One problem is the high volume of liquid required to conduct treatment. Patients typically keep a month's supply of dialysate solution on hand. This requires a large storage room and moving large amounts of liquid from the storage place to the treatment place. In addition, there is significant risk for infection. These procedures are done in the home by the patient or a helper to the patient that may not necessarily be well trained in aseptic techniques. As a result, introduction of bacteria due to the procedures causing an infection frequently occur.

There are several problems associated with the use of carbohydrate containing osmotic agents. For example, dextrose and glucose migrate through the peritoneum and into the blood stream resulting in elevated blood levels of these constituents. As a consequence, only relatively low concentrations of these osmotic agents can be used; thus, leading to the use of relatively large volumes of solutions and long dialysis times.

Another disadvantage associated with carbohydrate osmotic agents results from the common practice of using gamma radiation to sterilize the dialysis solution. Gamma radiation tends to degrade the osmotic agents yielding degradation products that lower the pH of the solution.

U.S. Patent 4,886,789 describes mixtures containing at least 15 weight percent of glucose polymers having a degree of polymerization greater than 12. Glucose polymers of this type can be relatively expensive to synthesize and are susceptible to degradation when subjected to gamma sterilization.

U.S. Patent 4,339,433 discloses a variety of dialysis solutions including non-carbohydrate osmotic agents. Unfortunately, these agents can be susceptible to degradation when subjected to gamma sterilization. Moreover, these agents are relatively expensive to produce.

Low cost, non-carbohydrate osmotic agents are sought which address the shortcomings associated with known osmotic agents. Moreover, a means for reducing the detrimental effects associated with radiation induced degradation products is sought.

This invention relates to a dialysis solution and method for its use wherein the solution includes an osmotic agent comprising a water soluble polyglycol having a molecular weight (number average molecular weight) from 500 to 20,000 daltons.

Selection of a specific polyglycol species may be at least partially based upon the pore size of the semipermeable membrane used in the dialysis treatment. The pore size of such membranes tends to be distribution of sizes rather than a uniform size. Nonetheless, such membranes are commonly characterized in terms of a "molecular weight cut-off' value. Materials having a molecular weight greater than the specified molecular weight cut-off of a membrane are substantially blocked, or incapable of passing through the membrane. For most applications, the subject polyglycols include polyethylene glycol having a molecular weight from: 3,000 to 5,000 for use in peritoneal dialysis and from 6,000 to 10,000 in hemodialysis utilizing currently marketed ultrafiltration membranes having a molecular weight cutoff of about 5,000. Lower molecular weight polyglycols, e.g., from 1,000 to 3,000, can be used in hemodialysis utilizing semipermeable membranes having very small molecular weight cutoffs (e.g., 500 to 1,000).

The subject osmotic agents are substantially impermeable through the peritoneum and the semipermeable membranes typically used in hemodialysis. Consequently, relatively high concentrations of the subject osmotic agent can be safely used, resulting in a significant reduction in the total volume of dialysis solution required and the time required for dialysis treatment. Moreover, many polyglycols suitable for use in the present invention are produced on a large commercial scale and are relatively inexpensive. For example, pharmaceutical grade polyethylene glycol having a range of suitable molecular weights are available.

Species of the subject polyglycols are well known for their biocompatibility and safety in medical uses, e.g., ophthalmic solutions, wound dressings, drug delivery, mucoadhesives, etc. Moreover, polyethylene glycol (e.g., polyethylene oxide) has been utilized in postoperative procedures for preventing adhesion formation, inflammatory reaction and collagen deposition within the peritoneum. See for example, Nagelschmidt, Manfred; Minor, Thomas; Saad, Stefan, "Polyethylene Glycol 4000 Attenuates Adhesion Formation in Rats by Suppression of Peritoneal Inflammation and Collagen Incorporation" *Am. J. Surg.* (1998), 176(1), 76-80.

It is a further aim of this invention to include free radical scavengers in the dialysis solution. In this manner, sterilization of the solution using gamma radiation can be accomplished with minimal damage to solution components while maintaining a physiological pH. One example of such a free radical scavenger is human serum albumin. The subject free radical scavengers can be utilized independently from the osmotic agents disclosed herein.

In several embodiments of the present invention, it is a further aim to include a polyglycol material having functional groups capable of forming associations with waste products (e.g., urea) removed from the blood during dialysis.

It is yet another aim of this invention to use a filter when introducing the solution in the peritoneal cavity to reduce the possibility of infection. Filters with a pore size of 0.2 microns are known to reject the passage of bacteria.

The dialysis solution of the present invention includes a physiologically acceptable aqueous solution including a water-soluble polyglycol. The solution has a physiologically acceptable pH and preferably includes physiologically acceptable salts, buffers and other constituents, as is well known in the art. For example, U.S. Patent 4,308,255 to Raj et al. describes dialysis solutions including physiologically acceptable quantities of sodium, chloride, potassium, bicarbonate, calcium, and magnesium.

The molecular weight of the subject polyglycol must be sufficiently high to prevent significant quantities of polyglycol from passing through the semipermeable membrane during dialysis treatment. However, the molecular weight must be low enough such that reasonable quantities of the polyglycol provide a sufficient osmotic driving force for water and waste products to pass through a semipermeable membrane and into the dialysis solution. Although dependant upon the specific composition and structure (e.g., linear, branched, etc.), the preferred molecular weight range for the subject polyglycols is from 500 to 20,000, depending at least partially upon the molecular weight cut-off of the membrane being utilized for dialysis. For most applications, molecular weights from about 3,000 to 5,000 are particularly preferred. It should be understood that higher molecular weight polyglycols may be used in combination with those falling within the specified range.

For purposes of this invention, the term "polyglycols" is intended to include water soluble polymers including repeating units represented by: -(CH₂CH₂O)-. Examples of such polyglycols include polyethylene glycol, also known as polyether glycol or polyoxyethylene. Preferred species included can be described as having the following repeating units: -(CH₂CH₂O)ₙ- wherein n is an integer resulting in a molecular weight within the range described above. In addition to linear polymers, the subject polyglycols may further include branched polymers including multiple branches of repeating units represented by -(CH₂CH₂O)-. Such polymers can be produced by polymerizing, grafting or otherwise reacting individual ethylene oxide groups, or polymers or pre-polymers thereof with polyhydric alcohols (e.g., glycerol, carbohydrates and the like), polyhydroxy aldehydes, polyhydroxy ketones, etc., such as by way of a condensation reaction via a pendant hydroxyl groups of glycerol, ribose etc. By way of further example, other compounds falling within the subject definition of polyglycols can be formed through use of polymers based on the monomer epichlorohydrin wherein the organic chloride groups of the resulting polymer are derivitized with amines to form quaternary amine groups. In light of the proceeding description those skilled in the art will readily appreciate alternative routes for making applicable polyglycols with the scope of the present invention. As compared to linear polymers such as polyethylene glycol, relatively smaller molecular weights of such branched polymers may be utilized, depending upon the specific configuration of the polymer and the pore size of the semipermeable membrane used therewith. That is, branched polymers may offer added steric hinderance such that relatively smaller molecular weight species will not pass through the pores of the semipermeable membrane used during dialysis.

The subject polyglycols may include terminal hydroxyl, aldehydes, carboxylic acid groups and/or other functional groups that are capable of forming association with blood borne waste products (e.g., urea). National formulary (NF) grades of these materials are preferred.

The subject polyglycols may be used in combination with other known osmotic agents glucose, dextrose, and other carbohydrate containing osmotic agents. Moreover, the subject polyglycols may be used in combination with the osmotic agents described in U.S. Patents 5,869,444; 4,761,237; 4,976,683; 4,604,379; 4,959,175; 4,339,433; and 4,886,789. However, the subject polyglycols preferably do not include "surfactants", (e.g., polysorbate); that is, they do not include significant hydrophilic portions which result in water insolubility.

Peritoneal dialysis solutions of the present invention typically include from 0.05 mole to 1.0 mole of the polyglycol per cycle of treatment. In the case where 1 or 2 liters of dialysate are used for one cycle of peritoneal dialysis, 100 to 10,000 grams of polyglycol per liter of solution are typically used, depending upon the specific application, the presence of other osmotic agents, and the molecular weight of the polyglycol. More typically, a dialysis cycle would use from 0.1 to 0.5 mole of polyglycol wherein the dialysis solution includes about 300 grams of polyglycol per liter of solution.

Hemodialysis solutions of the present solution typically include from 0.001 mole to 1.0 mole of the polyglycol per liter of solution, depending upon the specific application, the presence of other osmotic agents, the molecular weight of the polyglycol, and the desired mechanical pressure. More typically, 0.005 mole to 0.1 mole of polyglycol per liter of solution would be used.

The subject dialysis solution preferably includes a free radical scavenger to reduce complications caused by the production of degradation products from gamma sterilization. Examples of preferred free radical scavengers include: salicylic acid, Fe(II) phenantholine, dihydroxybenzoic acid (gentisic acid), human serum albumin, glutathione, and cysteine. Other examples include: ascorbic acid, benzyl alcohol, BHT, citric acid, glycerol, cysteamine, sulfarlem, tryptophan and iodoacetamide. It will be appreciated that the use of such free radical scavenger is independent of the specific osmotic agent and may be used with traditional, prior art, or non-polyglycol containing dialysis solutions.

In performing dialysis according to the present invention, risk of infection is minimized by including an in-line filter when introducing the present solution into the peritoneal cavity. Filters rejecting materials larger than 0.2 microns are well known to prevent the passage of bacteria.

The subject polyglycols are less susceptible to forming detrimental degradation products when exposed to sterilization conditions than conventional glucose solutions. By way of illustration, comparable dialysis solutions were prepared utilizing different osmotic agents: glucose and polyethylene glycol 3350 (approximate molecular weight). The solutions were subjected to common sterilization conditions, i.e., autoclave and gamma radiation (with Cobalt 60). After sterilization, the pH of each solution was measured. The pH of the glucose containing solutions had dropped significantly; whereas the pH of polyethylene glycol containing solutions had remained relatively stable.

### Specific Embodiments of the Invention

The following example illustrates the invention and should not be construed as limiting the scope of the appended claims.

### Example 1:

The following quantities of solutes were dissolved in 100 ML of water:
Calcium Chloride 0.039 g
Magnesium Chloride 0.014 g
Sodium Chloride 0.567 g
Sodium Lactate 0.392 g
Polyethylene glycol of 3,350 molecular weight, 29.14 g (osmotic agent)

1.5 ML of 1-131-radioiodinated polyetheramine of 3,500 molecular weight was added to 30 ML of this solution. Four male Sprague Dawley rats (150-175 g body weight) were injected with 3 ML of the radioactive solution into the peritoneal cavity. The rats were anesthetized after 30 minutes, 18 hours, 43 hours, 66 hours and 146 hours. A gamma camera was used to obtain an image of biodistribution of the animals at each time period. In addition, the amount of radioactive material left in the animals was determined by counting gamma emissions of the I-131 using the gamma camera. The images show that the radioactivity remained in the peritoneal cavity with no evidence of systemic uptake. The number of counts in the body as a function of time are consistent with little to no absorption of the polymer by the body.

## Claims

1. The use, in the preparation of a dialysis solution for use in dialysis, of an osmotic agent comprising a water soluble polyglycol having molecular weight from 500 to 20,000 daltons.

2. The use of Claim 1 wherein the osmotic agent has a molecular weight of from 3,000 to 5,000 daltons.

3. The use of Claim 1 wherein the osmotic agent comprises a linear polymer or containing repeating units represented by-(CH₂CH₂O)-.

4. The use of Claim 3 wherein the osmotic agent comprises polyethylene glycol.

5. The use of Claim 4 wherein the osmotic agent comprises polyethylene glycol having a molecular weight of about 3350.

6. The use of Claim 4 wherein the osmotic agent is polyethylene glycol having a molecular weight of about 4,000.

7. The use of Claim 1 wherein the osmotic agent comprises a branched polymer containing repeating units represented by- (CH₂CH₂O)-.

8. The use of Claim 7 wherein the osmotic agent comprises a branched polymer containing multiple branches including repeating units represented by -(CH₂CH₂O)-.

9. The use of any one of the preceding Claims, wherein the composition also comprises a free radical scavenger.

10. The use of Claim 9 wherein the free radical scavenger comprises serum albumin.

11. The use of Claim 9 wherein one or more free radical scavengers is used, selected from salicylic acid, Fe (II) phenantholine, dihydroxybenzoic acid, human serum albumin, glutathione, cysteine, ascorbic acid, benzyl alcohol, BHT, and citric acid.

12. The use of Claim 1 wherein the composition comprises from 0.05 mole to 1.0 mole of osmotic agent.

13. The use of Claim 9 wherein the composition comprises from 0.2 to 0. 5 mole of osmotic agent.

## Patentansprüche

1. Verwendung eines osmotischen Mittels, umfassend ein wasserlösliches Polyglykol mit einem Molekulargewicht von 500 bis 20 000 Dalton bei der Herstellung einer Dialyselösung zur Verwendung bei der Dialyse.

2. Verwendung nach Anspruch 1, worin das osmotische Mittel ein Molekulargewicht von 3 000 bis 5 000 Dalton besitzt.

3. Verwendung nach Anspruch 1, worin das osmotische Mittel ein lineares Polymer umfasst oder sich wiederholende Einheiten enthält, welche dargestellt sind durch -(CH₂CH₂O)-.

4. Verwendung nach Anspruch 3, worin das osmotische Mittel Polyethylenglykol umfasst.

5. Verwendung nach Anspruch 4, worin das osmotische Mittel Polyethylenglykol mit einem Molekulargewicht von etwa 3 350 umfasst.

6. Verwendung nach Anspruch 4, worin das osmotische Mittel Polyethylenglykol mit einem Molekulargewicht von etwa 4 000 ist.

7. Verwendung nach Anspruch 1, worin das osmotische Mittel ein verzweigtes Polymer umfasst, welches sich wiederholende Einheiten enthält, welche dargestellt sind durch -(CH₂CH₂O)-.

8. Verwendung nach Anspruch 7, worin das osmotische Mittel ein verzweigtes Polymer mit mehreren Verzweigungen umfasst, welches sich wiederholende Einheiten enthält, welche dargestellt sind durch -(CH₂CH₂O)-.

9. Verwendung nach einem der vorhergehenden Ansprüche, worin die Zusammensetzung auch einen Radikalfänger umfasst.

10. Verwendung nach Anspruch 9, worin der Radikalfänger Serumalbumin umfasst.

11. Verwendung nach Anspruch 9, worin ein oder mehrere Radikalfänger verwendet werden, ausgewählt aus der Gruppe Salicylsäure, Fe(II)-Phenantholin. Dihydroxybenzoesäure, humanem Serumalbumin, Glutathion, Cystein, Ascorbinsäure, Benzylalkohol, BHT und Citronensäure.

12. Verwendung nach Anspruch 1, worin die Zusammensetzung von 0,05 bis 1,0 Mol des osmotischen Mittels umfasst.

13. Verwendung nach Anspruch 9, worin die Zusammensetzung von 0,2 bis 0,5 Mol des osmotischen Mittels umfasst.

## Revendications

1. Utilisation dans la préparation d'une solution de dialyse pour l'utilisation dans la dialyse, d'un agent osmotique comprenant un polyglycol soluble dans l'eau ayant une masse moléculaire allant de 500 à 20 000 daltons.

2. Utilisation selon la revendication 1, dans laquelle l'agent osmotique a une masse moléculaire allant de 3 000 à 5 000 daltons.

3. Utilisation selon la revendication 1, dans laquelle l'agent osmotique comprend un polymère linéaire ou des motifs répétitifs représentés par -(CH₂CH₂O)-.

4. Utilisation selon la revendication 3, dans laquelle l'agent osmotique comprend du polyéthylène glycol.

5. Utilisation selon la revendication 4, dans laquelle l'agent osmotique comprend du polyéthylène glycol ayant une masse moléculaire d'environ 3350.

6. Utilisation selon la revendication 4, dans laquelle l'agent osmotique est du polyéthylène glycol ayant une masse moléculaire d'environ 4000.

7. Utilisation selon la revendication 1, dans laquelle l'agent osmotique comprend un polymère ramifié contenant des motifs répétitifs représentés par -(CH₂CH₂O)-.

8. Utilisation selon la revendication 7, dans laquelle l'agent osmotique comprend un polymère ramifié contenant des ramifications multiples qui comportent incluent des motifs répétitifs représentés par -(CH₂CH₂O)-.

9. Utilisation selon l'une des revendications précédentes dans laquelle la composition comprend également un désactiveur des radicaux libres.

10. Utilisation selon la revendication 9, dans laquelle le désactiveur des radicaux libres comprend de l'albumine sérique.

11. Utilisation selon la revendication 9, dans laquelle un ou plusieurs désactiveur des radicaux libres est utilisé, choisi parmi l'acide salicylique, le phénantholine de Fe (II), l'acide dihydroxybenzoïque, l'albumine sérique humaine, la glutathione, la cystéine, l'acide ascorbique, l'alcool benzylique, le BHT, et l'acide citrique.

12. Utilisation selon la revendication 1, dans laquelle la composition comprend entre 0,05 mole et 1,0 mole d'agent osmotique.

13. Utilisation selon la revendication 9, dans laquelle dans laquelle la composition comprend entre 0,2 mole et 0,5 mole d'agent osmotique.
